# EUROPÄISCHE PATENTSCHRIFT

(11) **EP 2 429 983 B1**
(45) Veröffentlichungstag und Bekanntmachung des Hinweises auf die Patenterteilung: **26.07.2017**
(21) Anmeldenummer: 10715897.4
(22) Anmeldetag: 05.05.2010
(51) Int. Cl.: C07C 209/36, C07C 211/46

(54) **VERFAHREN ZUR HERSTELLUNG VON ANILIN**
METHOD FOR PRODUCING ANILINE
PROCÉDÉ DE PRÉPARATION DE L'ANILINE

(30) Priorität: 14.05.2009 EP 09160263
(43) Veröffentlichungstag der Anmeldung: 21.03.2012
(73) Patentinhaber: BASF SE, 67056 Ludwigshafen am Rhein (DE)
(72) Erfinder: KÖNIGSMANN, Lucia, 70197 Stuttgart (DE); SCHWAB, Ekkehard, 67434 Neustadt (DE); HESSE, Michael, 67549 Worms (DE); SCHNEIDER, Christian, 68165 Mannheim (DE); HEIDEMANN, Thomas, 68519 Viernheim (DE); LIEKENS, Celine, B-2018 Antwerpen (BE); BICKELHAUPT, Jutta, 64407 Fränkisch-Crumbach (DE); THEIS, Dirk, 67069 Ludwigshafen (DE)
(86) Internationale Anmeldenummer: PCT/EP2010/056056
(87) Internationale Veröffentlichungsnummer: WO 2010/130604

(56) Entgegenhaltungen:
- DE-A1- 1 442 567
- DE-A1- 10 124 600
- DE-A1-102004 006 104
- DE-B- 1 127 904
- DE-B- 1 278 411

## Beschreibung

Die Erfindung betrifft ein Verfahren zur Herstellung von aromatischen Aminen, insbesondere Anilin, unter Verwendung von Kupfer-haltigen Katalysatoren auf SiO₂-Trägern.

Herstellungsverfahren von aromatischen Aminen wie Anilin durch Hydrierung der entsprechenden Nitroverbindungen sind bereits seit längerem bekannt. WO 2008/034770 betrifft ein Verfahren zur Herstellung von aromatischen Aminen in einem Wirbelschichtreaktor. Dabei durchströmt ein gasförmiges Reaktionsgemisch, enthaltend die Nitroverbindung und Wasserstoff, einen heterogenen partikelförmigen, eine Wirbelschicht ausbildenden Katalysator von unten nach oben. In der Wirbelschicht sind Einbauten vorgesehen, die wiederum den Katalysator enthalten, wobei die Einbauten eine spezielle Geometrie aufweisen. Als Katalysatoren können sowohl geträgerte als auch nicht geträgerte Katalysatoren eingesetzt werden, die Schwermetalle der 1. und/oder 5. bis 8. Gruppe des Periodensystems der Elemente (PSE) enthalten, insbesondere eines oder mehrere der Elemente Kupfer, Palladium, Molybdän, Wolfram, Nickel oder Kobalt. Hinsichtlich des optional enthaltenen Trägers sind jedoch keine zusätzlichen Informationen enthalten.

CN-A 1 657 162 betrifft einen Wirbelschichtkatalysator zur Herstellung von Anilin durch Hydrierung von Nitrobenzol. Der Katalysator enthält SiO₂ als Träger sowie Kupfer, Chrom, Molybdän sowie ein weiteres Metall ausgewählt aus Nickel, Zink, Barium, Vanadium, Bismuth, Blei oder Palladium. Die vorgenannten Metalle liegen als Oxid in einem speziellen Gewichtsverhältnis im Katalysator vor. Die vorgenannten Metalle werden bereits bei der Herstellung von SiO₂ in Form von wässrigen Salzlösungen in das Verfahren eingebracht, die Metalle werden hingegen nicht auf den bereits fertigen Träger aufgezogen.

Ein Verfahren zur Herstellung von aromatischen Aminen wie Anilin durch Hydrierung der entsprechenden Nitroaromaten mittels Wasserstoff an ortsfesten Katalysatoren unter adiabatischen Bedingungen ist in EP-A 1 882 681 beschrieben. Als Katalysatoren eignen sich prinzipiell alle für die Gasphasenhydrierung von Nitroverbindungen einsetzbaren Katalysatoren. Die Metallkomponente der Katalysatoren kann entweder in Form einer Legierung oder als Mischoxid vorliegen und der Katalysator kann gegebenenfalls unter Verwendung von einem inerten Trägermaterial hergestellt werden. Als Trägermaterialien kommen beispielsweise Aluminiumoxid (α- und γ-Modifikation), SiO₂, TiO₂, Roterde, Wasserglas oder Graphit in Frage. Als Metalle werden vorzugsweise

EP-A 1 935 871 sowie DE-A 10 2006 007 619 betreffen vordergründig die Aufreinigung von Anilin, das durch Hydrierung von Nitrobenzol in Gegenwart von Katalysatoren hergestellt wurde. Die Aufreinigung kann durch spezielle Destillationsverfahren oder Extraktion mit wässriger Alkalimetallhydroxid-Lösung erfolgen. Als Katalysatoren eignen sich beispielsweise solche, die Metalle der 8. Nebengruppe des PSE enthalten und die gegebenenfalls auf Trägermaterialien wie Kohlenstoff oder Oxiden von Magnesium, Aluminium und/oder Silicium aufgebracht sind. Geeignete Katalysatoren sind beispielsweise Raney-Kobalt- oder Raney-Nickel-Katalysatoren. Weiterhin sind ortsfeste, heterogene Trägerkatalysatoren wie Pd auf Aluminiumoxid oder Kohleträger geeignet.

CN-A 1 634 860 betrifft einen Gasverteiler im Wirbelbettreaktor, der bei der Synthese von Anilin in einen Wirbelbettreaktor eingebaut ist. Der Gasverteiler enthält verschiedene Strahlrichtungen, wobei die Hauptgaseinlassrohrleitung mit dem Kreuzmittelpunkt einer gekreuzten verzweigten Rohrleitung verbunden ist, während eine Ringrohrleitung zur Gasverteilung in 1 bis 10 Kreise aufgeteilt wird und an die verzweigte Rohrleitung angeschlossen ist. Im Wirbelbettreaktor wird ein Metallträgerkatalysator mit einer mittleren Korngröße von 45 bis 300 µm verwendet, wobei die statische Füllhöhe des Katalysators das Zwei- bis Zehnfache des Reaktordurchmessers beträgt. Der Katalysator enthält als Hauptaktivkomponente Kupfer, wobei SiO₂ oder Al₂O₃ als Träger verwendet werden.

Verfahren zur Herstellung von Trägermaterialien für Katalysatoren, beispielsweise von Siliciumdioxid (SiO₂), sind seit längerem bekannt (Sol-Gel-Prozess). Ein spezielles Herstellungsverfahren von geträgerten Katalysatoren ist in DE-A 10 2004 006 104 offenbart. In diesem Verfahren wird zunächst ein Hydrogel hergestellt, das anschließend zu feinpartikulärem Hydrogel vermahlen wird, woraus wiederum eine Slurry auf Basis des feinpartikulären Hydrogels erzeugt wird. Anschließend wird die Slurry sprühgetrocknet unter Erhalt des Katalysatorträgers. Auf den Katalysatorträger wird wenigstens ein Übergangsmetall und/oder wenigstens eine Übergangsmetallverbindung aufgebracht. Prinzipiell sind alle Übergangsmetalle geeignet, unter anderem auch Kupfer, Nickel, Platin, Palladium, Nickel, Eisen, Chrom oder Titan. Die Katalysatoren werden zur Polymerisation und/oder Copolymerisation von Olefinen eingesetzt, aus den Olefinen können wiederum Fasern, Folien und/oder Formkörper hergestellt werden. Die Verwendung der Katalysatoren zur Herstellung von Anilin in einem Hydrierungsverfahren ist in DE-A 10 2004 006 104 jedoch nicht beschrieben.

DE 1 442 567 beschreibt ein Verfahren zur Synthese von Anilin durch Hydrierung von Nitrobenzol in Gegenwart eines Kupfer-haltigen Katalysators, der auf Kieselsäure geträgert ist. Dabei wird eine Kieselsäuregallerte in einer Korundscheibenmühle gemahlen, dann in einen Trockenturm eingedüst, mittels Trockenluft getrocknet und mit einer Kupferlösung getränkt. Der Zusatz einer wässrigen Alkalihydroxid-Lösung wird nicht offenbart.

DE 1 278 411 offenbart ebenfalls ein Verfahren zur Herstellung von Anilin durch Hydrierung von Nitrobenzol in Gegenwart eines Kupfer-haltigen Katalysators mit einem Kieselsäure-Träger. Hierbei wird die Wasserglaslösung mit einer Kupferlösung gemischt, die Masse in einer Korundscheibenmühle gemahlen, in einen Trockenturm eingedüst und in dem Trockenturm mittels Trockenluft getrocknet. Der Zusatz einer Alkalihydroxid-Lösung wird hier jedoch nicht beschrieben.

DE 1 127 904 offenbart ein Verfahren eines für die Synthese von aromatischen Nitroverbindungen verwendbaren Kupferkatalysators durch Niederschlagen von Kupfer auf einem SiO₂-Träger. Dabei wird ein SiO₂-Hydrogel mit einer Kupfer(II)nitratlösung imprägniert, gerührt, filtriert, gewaschen und sprühgetrocknet. Der Zusatz einer Alkalihydroxid-Lösung wird hier ebenfalls nicht offenbart.

Die der vorliegenden Erfindung zugrunde liegende Aufgabe besteht in der Bereitstellung eines neuen, wirtschaftlichen Verfahrens zur Herstellung von aromatischen Aminen, insbesondere von Anilin.

Die Aufgabe wird gelöst durch ein Verfahren zur Herstellung von aromatischen Aminen durch katalytische Hydrierung der entsprechenden aromatischen Nitroverbindung, dadurch gekennzeichnet, dass a) das aromatische Amin Anilin ist und dass ein Kupfer-haltiger Katalysator mit einem Träger enthaltend SiO₂ eingesetzt wird, wobei das SiO₂ durch Nassmahlung und anschließender Sprühtrocknung hergestellt wurde undb) während oder im Anschluss an die Nassmahlung eine wässrige Alkalihydroxid-Lösung zugesetzt wird.

Das erfindungsgemäße Verfahren hat den Vorteil, dass Anilin, mit hoher Ausbeute und/oder hoher chemischer Reinheit hergestellt werden können. Weiterhin lässt sich das erfindungsgemäße Verfahren verfahrenstechnisch sehr einfach durchführen. Ein zusätzlicher Vorteil ist darin zu sehen, dass durch die Nassmahlung, die vor der Sprühtrocknung durchgeführt wird, ein Katalysatorträger hergestellt wird, der mechanisch sehr stabil ist. Die im erfindungsgemäßen Verfahren eingesetzten Katalysatoren zeichnen sich durch eine hohe Abriebfestigkeit aus. Eine hohe Abriebfestigkeit bewirkt wiederum eine verlängerte Katalysatorstandzeit. Der Katalysator kann also für einen längeren Zeitraum bei der Hydrierung von Nitrobenzol unter Erhalt von Anilin eingesetzt werden, wobei der Umsatz und/oder die Selektivität an Anilin über einen längeren Zeitraum konstant bleiben oder sich nur unwesentlich verringern.

Ein besonders abriebfester Katalysator wird hergestellt, sofern während oder im Anschluss an die Nassmahlung eine wässrige Alkalihydroxid-Lösung zugesetzt wird. Das dabei erhaltene Trägermaterial weist weniger Risse auf als Trägermaterial(partikel), bei dem keine Zugabe von wässriger Alkalihydroxid-Lösung erfolgt, sondern im Anschluss an die Nassmahlung ein Trocknungs- und/oder Calcinierungsschritt durchgeführt wird.

Nachfolgend wird das erfindungsgemäße Verfahren zur Herstellung von Anilin näher beschrieben.

Katalytische Hydrierung bedeutet im Rahmen der vorliegenden Erfindung die Umsetzung von Nitrobenzol mit Wasserstoff in Gegenwart eines Katalysators zu Anilin. Die katalytische Hydrierung kann dabei nach dem Fachmann bekannten Methoden durchgeführt werden. Beispielsweise kann der Druck 1 bis 50 bar, bevorzugt 2 bis 20 bar, besonders bevorzugt 2 bis 10 bar betragen. Die Hydrierungstemperatur beträgt beispielsweise 150 bis 400 °C, bevorzugt 200 bis 300 °C, besonders bevorzugt 270 bis 300 °C.

Der im erfindungsgemäßen Verfahren eingesetzte Katalysator enthält als (katalytisch aktive) Metall-Komponente Kupfer (Kupfer-haltiger Katalysator). Weiterhin basiert der Katalysator auf einem Träger enthaltend Siliciumdioxid (SiO₂). Das im Träger des Kupfer-haltigen Katalysators enthaltene SiO₂ wurde durch Nassmahlung und anschließende Sprühtrocknung hergestellt. In anderen Worten ausgedrückt bedeutet dies, dass bei der Herstellung des Trägers des Kupfer-haltigen Katalysators ein NassmahlungsSchritt durchgeführt wird. Im Anschluss an den Nassmahlungsschritt erfolgt ein Sprühtrocknungsschritt.

Der im erfindungsgemäßen Verfahren eingesetzte Katalysator kann Kupfer (nachfolgende Konzentrationsangabe berechnet als CuO) in beliebigen Konzentrationen enthalten. Vorzugsweise enthält der Katalysator Kupfer zu 15 bis 40 Gew.-%, insbesondere zu 25 bis 35 Gew.-%. Die Gewichtsprozent-Angaben an Kupfer sowie gegebenenfalls vorhandener weiterer Metalle beziehen sich auf das Katalysator-Gesamtgewicht, also unter Einbezug des Trägermaterials. Kupfer sowie gegebenenfalls vorhandene weitere Metalle können dabei in beliebigen Oxidationsstufen vorliegen, beispielsweise in der Oxidationsstufe 0 (metallisches Kupfer) und/oder in der Oxidationsstufe +I und/oder +II in Form von Kupferoxiden. Sinngemäßes gilt für die gegebenenfalls vorhandenen weiteren Metalle.

Als weitere Metalle kann der Kupfer-haltige Katalysator mindestens ein Metall (als reines Element oder in Form einer Verbindung, beispielsweise als Oxid) ausgewählt aus der Hauptgruppe I, der Hauptgruppe II, der Nebengruppe VI und der Nebengruppe II des PSE enthalten. Gegebenenfalls können auch weitere Metalle im Kupfer-haltigen Katalysator enthalten sein, die dem Fachmann aus Anilin-Herstellungsverfahren bekannt sind, beispielsweise Palladium, Nickel, Kobalt oder Platin. Vorzugsweise enthält der Kupfer-haltige Katalysator zusätzlich mindestens ein Metall ausgewählt aus Kalium (K), Natrium (Na), Barium (Ba), Chrom (Cr), Molybdän (Mo), Palladium (Pd), Zink (Zn), Wolfram (W), Nickel (Ni) oder Kobalt (Co). Besonders bevorzugt enthält der Kupfer-haltige Katalysator zusätzlich mindestens ein Metall ausgewählt aus Kalium, Barium oder Zink. Die zusätzlichen Metalle können im Kupfer-haltigen Katalysator in beliebigen Konzentrationen enthalten sein, vorzugsweise ist die Summe des Gewichts der weiteren Metallkomponenten jedoch kleiner als das Gewicht des im Katalysator enthaltenen Kupfers. Vorzugsweise beträgt die Summe der im Kupfer-haltigen Katalysator enthaltenen zusätzlichen Metalle 1 bis 10 Gew.-%, insbesondere 1 bis 5 Gew.-%.

Verfahren zur Herstellung von Kupfer-haltigen Katalysatoren sind dem Fachmann prinzipiell bekannt. Dabei können das Kupfer sowie gegebenenfalls die zusätzlich vorhandenen Metalle einerseits auf den fertigen Träger aufgebracht werden. Dies bedeutet, dass zunächst der Träger als solcher hergestellt wird, bevor Kupfer und gegebenenfalls mindestens ein weiteres Metall auf den fertigen Träger aufgebracht wird. Fertiger Träger im Rahmen der vorliegenden Erfindung bedeutet, dass die einzelnen Katalysatorpartikel bereits (nahezu) vollständig ausgebildet sind. Ein fertiger Träger liegt somit bereits nach dem Nassmahlschritt und anschließendem Sprühtrocknungsschritt vor. Das Aufbringen von Kupfer und/oder von weiteren Metallen erfolgt nach dem Trocknungs- und/oder Kalzinierungsschritt der fertigen Trägerpartikel.

Andererseits können Kupfer und/oder die weiteren Metalle auch bereits bei der Träger-herstellung in das Katalysator-Herstellungsverfahren eingeführt werden. Vorzugsweise wird der Kupfer-haltige Katalysator des erfindungsgemäßen Verfahrens hergestellt, indem Kupfer und gegebenenfalls mindestens ein weiteres Metall auf den fertigen Träger enthaltend SiO₂ aufgebracht wird. Methoden zum Aufbringen von Metallen wie Kupfer auf einen fertigen Träger sind dem Fachmann bekannt. Vorzugsweise erfolgt das Aufbringen durch Tränken des Trägers mit einer Lösung enthaltend Kupfer und gegebenenfalls mindestens ein weiteres Metall. Kupfer und die weiteren Metalle können beispielsweise als Carbonatlösungen, insbesondere ammoniakalische Carbonatlösungen, oder Nitratlösungen zugegeben werden. Die Zugabe kann portionsweise für jede Metallkomponente oder aber auch in einem Schritt erfolgen.

Vorzugsweise weist der Kupfer-haltige Katalysator eine Oberfläche von 150 bis 380 m²/g auf. Weiterhin ist es bevorzugt, den Kupfer-haltigen Katalysator als Wirbelschichtkatalysator (in einem Wirbelschichtreaktor) einzusetzen.

Der Kupfer-haltige Katalysator des erfindungsgemäßen Verfahrens enthält einen Träger, wobei der Träger wiederum SiO₂ enthält, das durch Nassmahlung und anschließender Sprühtrocknung hergestellt wurde. Nassmahlverfahren (Nassmahlungen) zur Herstellung von Partikeln einer bestimmten Größe sind dem Fachmann prinzipiell bekannt. Unter Nassmahlen im Sinne der vorliegenden Erfindung wird das Verkleinern des bereits gebildeten Siliciumdioxids (SiO₂) zu Partikeln einer bestimmten Größe/Durchmesser verstanden. Das Zerkleinern erfolgt durch Vermahlen in einer hierfür geeigneten Mühle, beispielsweise einer Stift- oder einer Pralltellermühle, insbesondere einer Rührwerkskugelmühle. Das zu zerkleinernde Siliciumdioxid wird dabei in einer Flüssigkeit vorgelegt. Das sich in der Flüssigkeit befindliche Siliciumdioxid wird auch als Maische bezeichnet. Vorzugsweise hat die Maische einen Feststoffgehalt von 10 bis 15 Gew.-% und einen pH-Wert von 5 bis 10. Vorzugsweise handelt es sich um eine SiO₂-Suspension, insbesondere um eine wässrige SiO₂-Suspension.

Das in der Maische bei der Nassmahlung enthaltene SiO₂ kann nach dem Fachmann bekannten Verfahren hergestellt werden. Vorzugsweise wird das SiO₂ nach dem Sol-Gel-Prozess hergestellt (Produktion von Silicagel). Beispielsweise kann Natriumsilikat wie Na₂SiO₃ mit Schwefelsäure zu SiO₂ umgesetzt werden. Das Reaktionsprodukt kann mit wässriger Ammoniaklösung und/oder mit in Wasser gelöstem Kohlendioxid gewaschen werden. Das dabei erhaltene Material ist grobkörnig und wird in der Regel anschließend einem Zerkleinerungsprozess, insbesondere einem Mahlprozess zugeführt. Im Rahmen der vorliegenden Erfindung erfolgt dies als Nassmahlung.

Die bei der Nassmahlung hergestellten SiO₂-Partikel können beliebige Größen/Durchmesser aufweisen. Vorzugsweise werden durch die Nassmahlung SiO₂-Partikel mit einem (Durchschnitts-)Durchmesser von 1 bis 35 µm, insbesondere 2 bis 30 µm hergestellt. Das bei der Nassmahlung und anschließender Sprühtrocknung hergestellte SiO₂ weist vorzugsweise eine Oberfläche von 400 bis 620 m²/g auf.

Vorzugsweise enthält der Träger des Kupfer-haltigen Katalysators mehr als 95 Gew.-%, insbesondere mehr als 98 Gew.-% SiO₂, bezogen auf den Träger als solchen ohne Berücksichtigung der katalytisch aktiven Metallkomponenten. Vorzugsweise ist der Träger frei oder weitgehend frei von Additiven und/oder Bindemitteln. Gegebenenfalls können jedoch zusätzliche Additive, Bindemittel oder sonstige Substanzen im Träger neben SiO₂ enthalten sein. Diese zusätzlichen Komponenten können entweder vor dem Nassmahlschritt, beispielsweise während des Sol-Gel-Prozesses, während oder auch nach dem Nassmahlschritt zugegeben werden. Gegebenenfalls kann der Kupfer-haltige Katalysator auch auf einem Trägergemisch enthaltend SiO₂ sowie weiteren dem Fachmann bekannten Trägermaterialien wie Aluminiumoxid eingesetzt werden.

Das bei dem Nassmahlschritt erhaltene SiO₂ kann gegebenenfalls direkt als Träger für den Kupfer-haltigen Katalysator verwendet werden. Normalerweise wird nach dem Nassmahlschritt ein zusätzlicher Sprühtrocknungsschritt und gegebenenfalls ein Calcinierungsschritt durchgeführt. Das Trocknen und/oder Calcinieren von SiO₂, das durch Mahlschritte, insbesondere Nassmahlschritte, erhalten wird, ist dem Fachmann bekannt. Vorzugsweise wird im Trocknungsschritt die aus der Maische stammende Flüssigkeit durch Versprühen entfernt. Vorzugsweise wird im Anschluss an den Trocknungsschritt ein Calcinierungsschritt durchgeführt. Calcinierungsschritte werden vorzugsweise bei Temperaturen von 150 bis 300 °C, insbesondere 250 bis 280 °C durchgeführt. Die Calcinierungsdauer beträgt normalerweise 1 bis 5 Stunden.

In der vorliegenden Erfindung wird dem SiO₂ während oder im Anschluss an die Nassmahlung eine wässrige Alkalihydroxidlösung zugesetzt. Vorzugsweise wird eine wässrige Natriumhydroxid (NaOH)-Lösung zugesetzt, beispielsweise in Form einer 25 %igen Lösung. Vorzugsweise wird dies im Anschluss an die Nassmahlung durchgeführt, also zu einer Maische, die bereits SiO₂-Partikel der gewünschten Größe enthält, vorzugsweise SiO₂-Partikel mit einem Durchmesser von 1 bis 35 µm. Insbesondere wird dieser Schritt kurz vor einem Trocknungsschritt durchgeführt, insbesondere ein Trocknungsschritt durch Versprühen der Maische-Flüssigkeit. In einer weiteren Ausführungsform der vorliegenden Erfindung werden mindestens zwei durch Nassmahlung erhaltene Fraktionen miteinander vermischt, wobei die beiden Fraktionen SiO₂-Partikel mit einem unterschiedlichen (Durchschnitts)durchmesser enthalten. Im Anschluss an den Mischvorgang kann beispielsweise die Zugabe einer wässrigen Alkalihydroxid-Lösung und/oder Trocknungs- sowie Calcinierungsschritte erfolgen.

In einer Ausführungsform der vorliegenden Erfindung kann der vorstehend beschriebene Kupfer-haltige Katalysator in einem Wirbelschichtreaktor eingesetzt werden, wobei in der Wirbelschicht Einbauten vorgesehen sind, die die Wirbelschicht in eine Mehrzahl von horizontal sowie eine Mehrzahl von vertikal im Wirbelschichtreaktor angeordneten Zellen aufteilen. Solche Wirbelschichtreaktoren sind beispielsweise in WO 2008/034770 beschrieben. Dabei wird ein gasförmiges Reaktionsgemisch, enthaltend die entsprechende Nitroverbindung und Wasserstoff, von unten nach oben über den Kupfer-haltigen Katalysator im Wirbelschichtreaktor geleitet. Die Zellwände der Zellen sind gasdurchlässig und weisen Öffnungen auf, die eine Austauschzahl des Kupfer-haltigen Katalysators in vertikaler Richtung im Bereich von 1 bis 100 Liter/h pro Liter Reaktorvolumen gewährleisten.

Vorzugsweise gewährleisten die Öffnungen in den Zellwänden der im Wirbelschichtreaktor angeordneten Zellen eine Austauschzahl des Kupfer-haltigen Katalysators in vertikale Richtung im Bereich von 10 bis 50 Liter/h pro Liter Reaktorvolumen und in horizontale Richtung von 0 oder im Bereich von 10 bis 50 Liter/h pro Liter Reaktorvolumen. Weiterhin ist es bevorzugt, die Einbauten als Kreuzkanalpackungen auszubilden mit in vertikaler Richtung im Wirbelbettreaktor parallel zueinander angeordneten geknickten gasdurchlässigen Metallblechen, Strickmetall- oder Gewebelagen, mit Knickkanten, die Knickflächen mit einem von 0 verschiedenen Neigungswinkel zur Vertikalen ausbilden, und wobei die Knickflächen aufeinanderfolgender Metallbleche, Strickmetall- oder Gewebelagen den gleichen Neigungswinkel, jedoch mit umgekehrtem Vorzeichen aufweisen und dadurch Zellen ausbilden, die in vertikaler Richtung durch Engstellen zwischen den Knickkanten begrenzt werden. Die Neigungswinkel der Druckflächen zur Vertikalen liegen im Bereich von 10 bis 80°, insbesondere zwischen 30 und 60°.

In einer weiteren Ausführungsform wird der Katalysator in einem Wirbelschichtreaktor eingesetzt wie beispielweise in DE-A 11 48 20 oder DE-A 11 33 394 beschrieben.

In einer weiteren Ausführungsform der vorliegenden Erfindung wird das Verfahren in einem Wirbelbettreaktor durchgeführt, in dem ein Gasverteiler eingebaut ist. Solche Gasverteiler sind dem Fachmann bekannt, sie sind beispielsweise in CN-A 1 634 860 beschrieben.

Die im erfindungsgemäßen Verfahren hergestellten aromatischen Amine können gegebenenfalls im Anschluss an die katalytische Hydrierung einem oder mehreren Aufreinigungsschritten unterzogen werden. Beispielsweise kann die Aufreinigung durch Destillation erfolgen. So kann das in der Reaktion vorhandene Wasser aus dem Reaktionsgemisch vom erhaltenen aromatischen Amin destillativ abgetrennt werden, wobei in einem einzigen Destillationsschritt der Wassergehalt des organischen Amins auf weniger als 20 Gew.-% bezogen auf das Gemisch aus organischem Amin und Wasser gesenkt werden kann. Die bei der Hydrierung freiwerdende Reaktionswärme kann zur Beheizung der vorstehend beschriebenen Destillation verwendet werden. Die Destillationskolonne wird vorzugsweise bei einem absoluten Kopfdruck von unter 1 bar betrieben. Darüber hinaus können auch eventuell anfallende Leichtsieder per Destillation abgetrennt werden.

Gegebenenfalls kann das bei der Hydrierung anfallende aromatische Amin (Roh-Amin) mit einer wässrigen Alkalimetallhydroxid-Lösung extrahiert und anschließend die wässrige und die organische Phase voneinander getrennt werden. Dabei werden die Konzentration der eingesetzten Alkalimetallhydroxid-Lösung und die Temperatur während der Extraktion so eingestellt, dass bei der Trennung der wässrigen und der organischen Phase die wässrige Phase die untere Phase darstellt.. Die Extraktion wird vorzugsweise bei Temperaturen von 30 bis 50 °C durchgeführt.

Die Erfindung wird anhand der nachfolgenden Beispiele weiter verdeutlicht.

### Beispiel 1: Träger-Herstellung

Ausgehend von Wasserglas und Schwefelsauere wird ein Kieselsäurehydrosol hergestellt unter Versprühung in einzelne Tropfen bei einer Gelbildungszeit von 1-15 Sekunden. Die Hydrosoltropfen nehmen dabei eine kugelartige Form an und werden in Wasser aufgenommen. Sie werden im Anschluss gewaschen. Die so erhaltener Hydrogelkugeln enthalten 14-20 Gew.-% SiO₂.

Die Hydrogelkugeln werden dann unter Zugabe von H₂O in einer Rührwerkskugelmühle zu einer Maische mit einem Feststoffgehalt von 13 Gew.-% vermahlen. Danach wird die Maische mit 25 % NaOH gemischt und im Anschluss sprühgetrocknet. Schließlich wird das so erhaltene Pulver bei 260-280°C für 4 h kalziniert. Die so hergestellten Partikel haben eine Microsphäroidale Form mit folgenden Merkmalen:
- Schüttdichte 470 kg/m³
- Partikelgrößen 10 -300µm
- BET Oberfläche 480 m²/g

### Beispiel 2: Herstellung eines Kupfer-haltigen Katalysators

60 g Träger aus Beispiel 1 werden im einen Rotationsverdampfer bei 120 °C Ölbadtemperatur vorgelegt. Zu dem Träger wird eine Mischung aus 179,02 g ammoniakalischer Kupfercarbonatlösung, 1,74 g Kaliumnitrat und 1,4 g Zinknitrat portionsweise zugegeben. Der hergestellte Katalysator wird getrocknet und im Anschluss bei 200-550 °C für 135 min calciniert (Katalysator 1).

Die analytischen Daten des Katalysators 1 sind in der folgenden Tabelle 2 zusammengefasst:

**Tabelle 2**

| | Feinanteil (%) | Abrieb (%) | BEToberfl. m2/g | Schüttdichte kg/m3 |
|---|---|---|---|---|
| Katalysator 1 | 4,3 | 8,8 | 375 | 790 |

Die Messung der Abriebsraten erfolgt in einer Strahlabriebsapparatur.
Der Abriebstest simuliert die mechanischen Belastungen, denen ein Wirbelgut (z.B. ein Katalysator) in einer Gas/Feststoff-Wirbelschicht ausgesetzt ist, und liefert eine Abriebsrate und einen Feinanteil, die das Festigkeitsverhalten beschreibt.

Die Abriebsapparatur besteht aus einem Düsenboden (Düsen-0 = 0,5mm), welcher mit einem Glasrohr gas- und feststoffdicht verbunden ist. Oberhalb des Glasrohrs ist ein Stahlrohr mit einer konischen Erweiterung ebenfalls gas- und feststoffdicht fixiert. Die Anlage ist an das 4-bar-Pressluftnetz angeschlossen. Ein Reduzierventil senkt den Druck vor der Anlage auf 2 bar absolut.

In die Anlage werden 60,0 g Katalysator eingefüllt. Die Pressluftmenge beträgt für die Versuchsdurchführung 350l/h. Die Anlage selbst wird unter atmosphärischen Bedingungen (1 bar, 20 °C) betrieben. Durch Partikel/Partikel- und Partikel/Wand-Stöße werden die Partikeln aufgrund der hohen Gasgeschwindigkeit an der Düse abgerieben bzw. zerkleinert. Der ausgetragene Feststoff gelangt über einen Rohrbogen in eine Hülse aus Filterpapier (Porenweite 10-15 µm) und das gereinigte Gas strömt in das Abluftsystem des Labors.

Der abgeschiedene Feststoff (Partikel < 20 µm) wird nach einer Stunde (definiert als Feinanteil) und nach 5 Stunden (definiert als Abrieb) gewogen.

### Beispiel 3: Anilin-Herstellung

Die Performance des Katalysators 1 wird im kontinuierlichen Betrieb geprüft in einem 5L Reaktor [T=290 °C, p=6bar absolut, Wasserstoff: 2 Nm3/h H_{2,} N₂: 8 Nm3/h, Katalysator Belastung 0,91 kg_{MNB}/kg_{Kat} x h]. Vorgewärmtes Nitrobenzol wird mittels einer Zweistoffdüse in den Reaktor gepumpt und dort mit einem Teil des Wasserstoff-Stromes an der Düsenöffnung vernebelt.
Mit dem Katalysator 1 wurde ein Umsatz von 100 % und eine Selektivität > 99,5 % über 11 Zyklen insgesamt 500h erzielt. Nach 11 Zyklen wurde die Reaktion unterbrochen, der Katalysator war jedoch weiterhin aktiv.

## Patentansprüche

1. Verfahren zur Herstellung von aromatischen Aminen durch katalytische Hydrierung der entsprechenden aromatischen Nitroverbindung, **dadurch gekennzeichnet, dass** das aromatische Amin Anilin ist und dass ein Kupfer-haltiger Katalysator mit einem Träger enthaltend SiO₂ eingesetzt wird, wobei das SiO₂ durch Nassmahlung und anschließender Sprühtrocknung hergestellt wurde und während oder im Anschluss an die Nassmahlung eine wässrige Alkalihydroxid-Lösung zugesetzt wird.

2. Verfahren gemäß Anspruch 1, **dadurch gekennzeichnet, dass** der Kupfer-haltige Katalysator Kupfer zu 15 bis 40 Gew.-% enthält.

3. Verfahren gemäß Anspruch 1 oder 2, **dadurch gekennzeichnet, dass** der Kupfer-haltige Katalysator zusätzlich mindestens ein Metall ausgewählt aus Kalium, Natrium, Barium, Chrom, Molybdän, Palladium, Zink, Wolfram, Nickel oder Kobalt enthält.

4. Verfahren gemäß einem der Ansprüche 1 bis 3, **dadurch gekennzeichnet, dass** der Kupfer-haltige Katalysator eine Oberfläche von 150 bis 380 m²/g aufweist.

5. Verfahren gemäß einem der Ansprüche 1 bis 4, **dadurch gekennzeichnet, dass** der Kupfer-haltige Katalysator als Wirbelschichtkatalysator eingesetzt wird.

6. Verfahren gemäß einem der Ansprüche 1 bis 5, **dadurch gekennzeichnet, dass** bei der Nassmahlung SiO₂-Partikel mit einem Durchmesser von 1 bis 35 µm hergestellt werden.

7. Verfahren gemäß einem der Ansprüche 1 bis 6, **dadurch gekennzeichnet, dass** bei der Nassmahlung eine Maische mit einem Feststoffgehalt von 10 bis 15 Gew.-% eingesetzt wird.

8. Verfahren gemäß einem der Ansprüche 1 bis 7, **dadurch gekennzeichnet, dass** während oder im Anschluss an die Nassmahlung eine wässrige NaOH-Lösung zugesetzt wird.

9. Verfahren gemäß einem der Ansprüche 1 bis 8, **dadurch gekennzeichnet, dass** das durch die Nassmahlung und anschließender Sprühtrocknung hergestellte SiO₂ eine Oberfläche von 400 bis 620 m²/g aufweist.

10. Verfahren gemäß einem der Ansprüche 1 bis 9, **dadurch gekennzeichnet, dass** Kupfer und gegebenenfalls mindestens ein weiteres Metall auf den fertigen Träger enthaltend SiO₂ aufgebracht wird.

11. Verfahren gemäß Anspruch 10, **dadurch gekennzeichnet, dass** das Aufbringen durch Tränken des Trägers mit einer Lösung enthaltend Kupfer und gegebenenfalls mindestens ein weiteres Metall erfolgt.

## Claims

1. A process for preparing aromatic amines by catalytically hydrogenating the corresponding aromatic nitro compound, wherein the aromatic amine is aniline and which comprises using a copper catalyst with a support comprising SiO₂, the SiO₂ having been prepared by wet grinding and subsequent spray drying and an aqueous alkali metal hydroxide solution being added during or after the wet grinding.

2. The process according to claim 1, wherein the copper catalyst comprises copper to an extent of 15 to 40% by weight.

3. The process according to claim 1 or 2, wherein the copper catalyst additionally comprises at least one metal selected from potassium, sodium, barium, chromium, molybdenum, palladium, zinc, tungsten, nickel or cobalt.

4. The process according to any of claims 1 to 3, wherein the copper catalyst has a surface area of 150 to 380 m²/g.

5. The process according to any of claims 1 to 4, wherein the copper catalyst is used as a fluidized bed catalyst.

6. The process according to any of claims 1 to 5, wherein SiO₂ particles with a diameter of 1 to 35 µm are prepared in the wet grinding.

7. The process according to any of claims 1 to 6, wherein a slurry with a solids content of 10 to 15% is used in the wet grinding.

8. The process according to any of claims 1 to 7, wherein an aqueous NaOH solution is added during or after the wet grinding.

9. The process according to any of claims 1 to 8, wherein the SiO₂ prepared by the wet grinding and subsequent spray drying has a surface area of 400 to 620 m²/g.

10. The process according to any of claims 1 to 9 wherein copper and optionally at least one further metal are applied to the finished support comprising SiO₂.

11. The process according to claim 1, wherein the application is effected by impregnating the support with a solution comprising copper and optionally at least one further metal.

## Revendications

1. Procédé pour la préparation d'amines aromatiques par hydrogénation catalytique du composé nitré aromatique correspondant, **caractérisé en ce que** l'amine aromatique est l'aniline et **en ce que** ce qu'on utilise un catalyseur contenant du cuivre avec un support contenant du SiO₂, le SiO₂ ayant été préparé par broyage par voie humide et séchage subséquent par atomisation et pendant ou à la suite du broyage par voie humide on ajoute une solution aqueuse d'un hydroxyde de métal alcalin.

2. Procédé selon la revendication 1, **caractérisé en ce que** le catalyseur contenant du cuivre contient du cuivre à raison de 15 à 40 % en poids.

3. Procédé selon la revendication 1 ou 2, **caractérisé en ce que** le catalyseur contenant du cuivre contient en outre au moins un métal choisi parmi le potassium, le sodium, le baryum, le chrome, le molybdène, le palladium, le zinc, le tungstène, le nickel ou le cobalt.

4. Procédé selon l'une quelconque des revendications 1 à 3, **caractérisé en ce que** le catalyseur contenant du cuivre présente une surface de 150 à 380 m²/g.

5. Procédé selon l'une quelconque des revendications 1 à 4, **caractérisé en ce que** le catalyseur contenant du cuivre est utilisé sous forme de catalyseur en couche tourbillonnaire.

6. Procédé selon l'une quelconque des revendications 1 à 5, **caractérisé en ce que** dans le broyage par voie humide on utilise des particules de SiO₂ ayant un diamètre de 1 à 35 µm.

7. Procédé selon l'une quelconque des revendications 1 à 6, **caractérisé en ce que** dans le broyage par voie humide on utilise une suspension ayant une teneur en matières solides de 10 à 15 % en poids.

8. Procédé selon l'une quelconque des revendications 1 à 7, **caractérisé en ce que** pendant le ou à la suite du broyage par voie humide on ajoute une solution aqueuse de NaOH.

9. Procédé selon l'une quelconque des revendications 1 à 8, **caractérisé en ce que** le SiO₂ préparé par le broyage par voie humide et séchage subséquent par atomisation présente une surface de 400 à 620 m²/g.

10. Procédé selon l'une quelconque des revendications 1 à 9, **caractérisé en ce qu'**on applique du cuivre et éventuellement au moins un autre métal sur le support prêt, comportant du SiO₂.

11. Procédé selon la revendication 10, **caractérisé en ce que** l'application s'effectue par imprégnation du support avec une solution contenant du cuivre et éventuellement au moins un autre métal.
